# EUROPEAN PATENT APPLICATION

(11) **EP 4 332 078 A1**
(43) Date of publication of application: **06.03.2024**
(21) Application number: 22795505.1
(22) Date of filing: 30.03.2022
(51) Int. Cl.: C07C 17/25, C07C 21/22, C09K 13/00

(54) **METHOD FOR PRODUCING FLUOROALKYNE COMPOUND**

(30) Priority: 27.04.2021 JP 2021075321
(71) Applicant: DAIKIN INDUSTRIES, LTD., Osaka-Shi, Osaka 530-0001 (JP)
(72) Inventor: ETOU, Yuusuke, Osaka-shi, Osaka 530-0001 (JP); NAKAMURA, Shingo, Osaka-shi, Osaka 530-0001 (JP)
(74) Representative: Hoffmann Eitle
(86) International application number: PCT/JP2022/016193
(87) International publication number: WO 2022/230589

(57) **Abstract**

A fluoroalkyne compound can be efficiently synthesized from a fluoroalkane compound by a method for producing a fluoroalkyne compound represented by formula (1):

R¹C≡CR² (1)

wherein R¹ and R² are the same or different and each is a fluorine atom or a fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of an ether solvent,
the method satisfying at least one requirement selected from the group consisting of the following:
(I) the fluoroalkyl group is a C₁-C₄ fluoroalkyl group, and
(II) the ether solvent is a chain ether compound, and the dehydrofluorination reaction is performed in the presence of the solvent containing a chain ether and a base comprising a hydroxide and/or alkoxide of an alkali metal and/or alkaline earth metal.

## Description

### Technical Field

The present disclosure relates to a method for producing a fluoroalkyne compound.

### Background Art

Fluoroalkyne compounds are expected to be useable as cleaning gases, etching gases, refrigerants, heat transfer media, building blocks for organic synthesis, etc.

As an example of a method for producing a fluoroalkyne compound, Non-patent Literature (NPL) 1 discloses that a fluoroalkyne compound is obtained from a fluoroalkane compound by a dehydrofluorination reaction, using a base such as lithium diisopropylamide (LDA).

### Citation List

### Non-patent Literature

NPL 1: Tetrahedron, 1988, vol. 44, No. 10, p. 2865-2874

### Summary of Invention

### Technical Problem

An object of the present disclosure is to provide a method for efficiently synthesizing a fluoroalkyne compound from a fluoroalkane compound.

### Solution to Problem

The present disclosure includes the following subject matter.

Item 1. A method for producing a fluoroalkyne compound represented by formula (1):

R¹C≡CR² (1)

wherein R¹ and R² are the same or different and each is a fluorine atom or a fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of a solvent containing an ether,
the method satisfying at least one requirement selected from the group consisting of the following:
(I) the fluoroalkyl group is a C₁-C₄ fluoroalkyl group, and
(II) the ether is a chain ether, and the dehydrofluorination reaction is performed in the presence of the solvent containing a chain ether, and a base comprising a hydroxide and/or alkoxide of an alkali metal and/or alkaline earth metal.

Item 2. The production method according to Item 1, wherein the water concentration of the solvent is 0.01 to 500 mass ppm based on the total amount of the solvent taken as 100 mass%.

Item 3. The production method according to Item 1 or 2, wherein the number of ether bonds in the ether is 1 to 10.

Item 4. The production method according to any one of Items 1 to 3, which satisfies requirement (I).

Item 5. The production method according to Item 4, wherein the fluoroalkyl group is represented by formula (3):

-CF₂R³ (3)

wherein R³ is a fluorine atom or a C₁-C₃ fluoroalkyl group.

Item 6. The production method according to Item 4 or 5, wherein the dehydrofluorination reaction is performed in the presence of a base.

Item 7. The production method according to Item 6, wherein the base is a hydroxide and/or alkoxide of an alkali metal and/or alkaline earth metal.

Item 8. The production method according to any one of Items 1 to 3, which satisfies requirement (II).

Item 9. The production method according to Item 8, wherein the fluoroalkyl group has 1 to 10 carbon atoms.

Item 10. The production method according to Item 8 or 9, wherein the fluoroalkyl group is represented by formula (3):

-CF₂R³ (3)

wherein R³ represents a fluorine atom or a C₁-C₉ fluoroalkyl group.

Item 11. The production method according to any one of Items 1 to 10, wherein the reaction temperature in the dehydrofluorination reaction is 0 to 300°C.

Item 12. A composition comprising a fluoroalkyne compound represented by formula (1):

R¹C≡CR² (1)

wherein R¹ and R² are the same or different and each is a fluorine atom or a fluoroalkyl group, and a fluoroalkene compound represented by formula (4):

R¹CF=CHR² (4)

wherein R¹ and R² are as defined above,
the content of the fluoroalkyne compound being 25.00 to 75.00 mol%, the content of the fluoroalkene compound being 25.00 to 75.00 mol%, and the total content of the fluoroalkyne compound and the fluoroalkene compound being 90.00 to 100.00 mol%, based on the total amount of the composition taken as 100 mol%.

Item 13. The composition according to Item 12, which is for use as a cleaning gas, an etching gas, a refrigerant, a heat transfer medium, or a building block for organic synthesis.

Item 14. Use of the composition according to Item 12 for a cleaning gas, an etching gas, a refrigerant, a heat transfer medium, or a building block for organic synthesis.

### Advantageous Effects of Invention

The present disclosure provides a method for efficiently synthesizing a fluoroalkane compound from a fluoroalkane compound.

### Description of Embodiments

In the present specification, the terms "comprise," "contain," and "include" encompass the concepts of comprising, consisting essentially of, and consisting of. In the present specification, a numerical range indicated by "A to B" means A or more, and B or less.

In the present disclosure, "selectivity" refers to the ratio (mol%) of the total molar amount of the target compound contained in a gas flowing out from a reactor outlet to the total molar amount of the compounds other than the starting compound in the gas flowing out from the reactor outlet.

In the present disclosure, "conversion" refers to the ratio (mol%) of the total molar amount of the compounds other than the starting compound contained in a gas flowing out from a reactor outlet to the molar amount of the starting compound supplied to the reactor.

In the present disclosure, "yield" refers to the ratio (mol%) of the total molar amount of the target compound contained in a gas flowing out from a reactor outlet to the molar amount of the starting compound supplied to the reactor.

A known method for producing a fluoroalkyne compound is a method in which a fluoroalkene compound, such as CF₃CF=CHCF₃, is synthesized from a fluoroalkane compound, such as CF₃CFHCFHCF₃, by a dehydrofluorination reaction, and then a dehydrofluorination reaction is further performed to synthesize a fluoroalkyne compound. In this method, it is necessary to perform a dehydrofluorination reaction twice, which lengthens the production process.

Another known method is a method in which a fluoroalkyne compound is obtained from a fluoroalkane compound by a single dehydrofluorination reaction using lithium diisopropylamide (LDA) as a base in a solvent such as hexane, diethyl ether, or monoethylene glycol dimethyl ether, as in NPL 1. In this method, a fluoroalkyne compound is obtained from a fluoroalkane compound by a single dehydrofluorination reaction using KOC(CH₃)₃ as a base, even in a solvent such as tetrahydrofuran or dimethyl sulfoxide. However, this method is not efficient when lithium diisopropylamide (LDA) is used in this method, because lithium diisopropylamide (LDA) is expensive, and the yield is at most 41% (at most 24% when expensive LDA is not used). Thus, this method is not an effective production method.

According to the production method of the present disclosure, a method for efficiently synthesizing a fluoroalkane compound from a fluoroalkane compound in a high yield compared with the conventional methods can be provided.

### 1. Method for Producing Fluoroalkyne Compound (First Embodiment)

The method for producing a fluoroalkyne compound in the first embodiment of the present disclosure is a method for producing a fluoroalkyne compound represented by formula (1):

R²C≡CR² (1)

wherein R¹ and R² are the same or different and each is a fluorine atom or a fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of a solvent containing an ether,
the method satisfying the following:
(I) the fluoroalkyl group is a C₁-C₄ fluoroalkyl group.

That is, the method for producing a fluoroalkyne compound in the first embodiment of the present disclosure is a method for producing a fluoroalkyne compound represented by formula (1):

R¹C≡CR² (1)

wherein R¹ and R² are the same or different and each is a fluorine atom or a C₁-C₄ fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of an ether solvent.

According to the present disclosure, by performing a dehydrofluorination reaction of the fluoroalkane compound represented by formula (2), the fluoroalkyne compound represented by formula (1) in which 2 mol of hydrogen fluoride is eliminated per mol of the fluoroalkane compound represented by formula (2) can be obtained in only a single step.

### (1-1) Starting Compound (Fluoroalkane Compound)

The fluoroalkane compound as a substrate that can be used in the production method of the present disclosure (the first embodiment) is a fluoroalkane compound represented by formula (2):

R¹CHX¹CFX²R² (2)

wherein R¹ and R² are the same or different and each is a fluorine atom or a C₁-C₄ fluoroalkyl group; X¹ is a fluorine atom and X² is a hydrogen atom, or X¹ is a hydrogen atom and X² is a fluorine atom, as described above.

That is, the fluoroalkane compound represented by formula (2) encompasses formulas (2A) and (2B):

R¹CFHCFHR² (2A)

R¹CH₂CF₂R² (2B)

wherein R¹ and R² are the same or different and each is a fluorine atom or a C₁-C₄ fluoroalkyl group.

In formula (2), the fluoroalkyl group represented by each of R¹ and R² refers to a group formed by replacing one or more hydrogen atoms of an alkyl group with a fluorine atom, and also includes a perfluoroalkyl group formed by replacing all the hydrogen atoms of an alkyl group. The fluoroalkyl group may be a linear fluoroalkyl group or a branched fluoroalkyl group. Examples include C₁-C₄ fluoroalkyl groups, such as monofluoromethyl, difluoromethyl, trifluoromethyl, monofluoroethyl, difluoroethyl, trifluoroethyl, tetrafluoroethyl, pentafluoroethylfluoroethyl, monofluoropropyl, difluoropropyl, trifluoropropyl, tetrafluoropropyl, pentafluoropropyl, hexafluoropropyl, heptafluoropropyl, monofluorobutyl, difluorobutyl, trifluorobutyl, tetrafluorobutyl, heptafluorobutyl, hexafluorobutyl, heptafluorobutyl, octafluorobutyl, and nonafluorobutyl. From the viewpoint of producing the fluoroalkyne compound in particular with high conversion, yield, and selectivity, the fluoroalkyl group is preferably a C₁-C₃ fluoroalkyl group. In addition, from the viewpoint of easily suppressing side reactions, the fluoroalkyl group preferably contains as few hydrogen atoms as possible, and is particularly preferably a perfluoroalkyl group.

In the fluoroalkane compound as a substrate, R¹ and R² are preferably fluoroalkyl groups, more preferably perfluoroalkyl groups, and even more preferably trifluoromethyl groups, from the viewpoint of producing the fluoroalkyne compound in particular with high conversion, yield, and selectivity.

R¹ and R² may be the same or different.

Specific examples of the fluoroalkane compound as a substrate that satisfies the above conditions include CF₂HCF₂H, CF₃CFHCF₂H, CF₃CFHCFHCF₃, CF₃CF₂CFHCFHCF₃, CF₃CF₂CFHCFHCF₂CF₃, CFH₂CF₃, CF₃CH₂CF₃, CF₃CH₂CF₂CF₃, CF₃CF₂CH₂CF₂CF₃, CF₃CF₂CH₂CF₂CF₂CF₃, and the like. These fluoroalkane compounds can be used singly or in a combination of two or more. Such fluoroalkane compounds can be known or commercially available products.

### (1-2) Dehydrofluorination Reaction

In the step of subjecting the fluoroalkane compound to a dehydrofluorination reaction in the present disclosure, for example, in the fluoroalkane compound represented by formula (2) as a substrate, R¹ and R² are more preferably trifluoromethyl groups.

That is, this reaction is preferably a dehydrofluorination reaction in which 2 mol of hydrogen fluoride is eliminated per mol of CF₃CFHCFHCF₃ or CF₃CH₂CF₂CF₃ according to the following reaction formulas:

CF₃CFHCFHCF₃ → CF₃C≡CCF₃ + 2HF

CF₃CH₂CF₂CF₃ → CF₃C≡CCF₃ + 2HF

The step of subjecting the fluoroalkane compound to a dehydrofluorination reaction in the present disclosure is performed in the presence of a solvent containing an ether, that is, in a liquid phase using a solvent containing an ether, from the viewpoint of conversion, selectivity, and yield. If the reaction is performed in a gas phase that does not use a solvent or in a liquid phase using a solvent other than a solvent containing an ether, the dehydrofluorination reaction of the fluoroalkene compound does not proceed, and the fluoroalkyne compound cannot be efficiently obtained from the fluoroalkane compound in a single step.

In the present disclosure, the yield of the target compound can be further improved, for example, by using a metal container, and increasing the amount of the liquid phase by applying pressure and raising the boiling point of the starting material.

Moreover, in the present disclosure, it is preferable to first prepare a solution of the fluoroalkane compound represented by formula (2), and then allow the reaction to proceed in the presence of a base.

### (1-3) Solvent Containing Ether

In the present disclosure, in order to obtain the fluoroalkyne compound from the fluoroalkane compound by a dehydrofluorination reaction, preferably in a single step, the reaction is performed in the presence of a solvent containing an ether.

However, the water content is preferably small so that the fluoroalkyne compound can be easily obtained from the fluoroalkane compound by a dehydrofluorination reaction. From such a viewpoint, the water concentration of the solvent containing an ether is preferably 500 mass ppm or less, more preferably 400 mass ppm or less, and even more preferably 300 mass ppm or less, based on the total amount of the solvent containing an ether taken as 100 mass%. The lower limit of the water concentration of the solvent containing an ether is not limited, and is preferably 0.01 mass ppm or more from the viewpoint that it can be easily achieved technically.

To easily obtain the fluoroalkyne compound from the fluoroalkane compound by a dehydrofluorination reaction, the number of ether bonds in the ether is preferably as small as possible. From such a viewpoint, the number of ether bonds in the ether is preferably 1 to 10, more preferably 1 to 5, even more preferably 1 to 3, and particularly preferably 1 to 2.

The ether in the solvent containing an ether that satisfies the above conditions is preferably a chain ether, from the viewpoint of conversion, selectivity, and yield. The chain ether includes not only an ether compound having no cyclic structure, but also an ether compound containing a cyclic structure that does not contain an ether bond. That is, the chain ether is a concept that does not include an ether compound in which an ether bond constitutes a cyclic structure, such as tetrahydrofuran, but includes an ether compound containing a cyclic structure that does not contain an ether bond, such as diphenyl ether. When an ether compound having no cyclic structure is used, the yield of the fluoroalkyne compound as the target product can be particularly improved. When an ether compound containing a cyclic structure that does not contain an ether bond is used, the yield of impurities (hydrofluorocarbon compounds other than fluoroalkane compounds, fluoroalkene compounds, and fluoroalkyne compounds) can be particularly reduced. Specific preferred examples of such ethers include diethyl ether, diisopropyl ether, di(n-butyl) ether, diphenyl ether, 1,2-dimethoxyethane, 1,2-diethoxyethane, diglyme, and the like. The ether solvents can be used singly or in a combination of two or more. In particular, from the viewpoint of conversion, selectivity, and yield, diethyl ether, diisopropyl ether, di(n-butyl) ether, diphenyl ether, 1,2-dimethoxyethane, 1,2-diethoxyethane, and the like are preferred, diethyl ether, diisopropyl ether, di(n-butyl) ether, diphenyl ether, and the like are more preferred, and diisopropyl ether, di(n-butyl) ether, diphenyl ether, and the like are even more preferred.

In the present disclosure, the ether may be used in combination with other solvents, such as carbonate ester solvents such as dimethyl carbonate, ethyl methyl carbonate, diethyl carbonate, methyl propyl carbonate, and ethyl propyl carbonate; ester solvents such as ethyl acetate, propyl acetate, butyl acetate, methyl propionate, ethyl propionate, and butyl propionate; ketone solvents such as acetone, ethyl methyl ketone, and diethyl ketone; lactone solvents such as γ-butyrolactone, γ-valerolactone, tetrahydrofuran, and tetrahydropyran; cyclic ether solvents such as tetrahydrofuran; nitrile solvents such as acetonitrile, propionitrile, and benzonitrile; amide solvents such as N,N-dimethylformamide; and sulfone solvents such as dimethyl sulfoxide and sulfolane. However, from the viewpoint of, for example, the conversion in reaction, selectivity, and yield, the amount of the ether for use is preferably 80 to 100 vol% (in particular 90 to 100 vol%), and the amount of other solvents is preferably 0 to 20 vol% (in particular 0 to 10 vol%), based on the total amount of the solvents for use in reaction taken as 100 vol%.

### (1-4) Base

From the viewpoint of conversion, selectivity, and yield, the step of subjecting a fluoroalkane compound to a dehydrofluorination reaction to obtain a fluoroalkyne compound in the present disclosure is preferably performed in the presence of a base.

From the viewpoint of the conversion in reaction and the selectivity and yield of the halogenated alkyne compound, the base is preferably at least one member selected from the group consisting of hydroxides and alkoxides of alkali metals and alkaline earth metals (i.e., at least one member selected from the group consisting of alkali metal hydroxides, alkaline earth metal hydroxides, alkali metal alkoxides, and alkaline earth metal alkoxides), more preferably at least one member selected from the group consisting of hydroxides of alkali metals and alkaline earth metals and alkoxides of alkali metals (i.e., at least one member selected from the group consisting of alkali metal hydroxides, alkaline earth metal hydroxides, and alkali metal alkoxides), even more preferably a hydroxide and/or alkoxide of an alkali metal (i.e., an alkali metal hydroxide and/or an alkali metal alkoxide), and particularly preferably an alkali metal hydroxide. Specific examples of bases include sodium hydroxide, potassium hydroxide, magnesium hydroxide, calcium hydroxide, sodium methoxide, potassium tert-butoxide, sodium ethoxide, sodium tert-butoxide, potassium methoxide, potassium ethoxide, potassium tert-butoxide, magnesium methoxide, magnesium ethoxide, magnesium tert-butoxide, calcium methoxide, calcium ethoxide, calcium tert-butoxide, and the like. In particular, from the viewpoint of the conversion in reaction and the selectivity and yield of the halogenated alkyne compound, sodium hydroxide, potassium hydroxide, magnesium hydroxide, calcium hydroxide, sodium methoxide, sodium ethoxide, sodium tert-butoxide, potassium methoxide, potassium ethoxide, potassium tert-butoxide, and the like are preferred, sodium hydroxide, potassium hydroxide, sodium methoxide, sodium ethoxide, sodium tert-butoxide, potassium methoxide, potassium ethoxide, potassium tert-butoxide, and the like are more preferred, and sodium hydroxide, potassium hydroxide, and the like are even more preferred.

The amount of the base for use is not limited, and is preferably 1.0 to 10.0 mol, more preferably 1.5 to 5.0 mol, and even more preferably 2.0 to 3.0 mol, per mol of the fluoroalkane compound represented by formula (2).

### (1-5) Reaction Conditions

### Closed Reaction Process

In the present disclosure, the fluoroalkyne compound represented by formula (1) as the target compound has a low boiling point and exists as a gas at room temperature. Accordingly, in the step of performing a dehydrofluorination reaction in the present disclosure, when a closed reaction process is used as the reaction system, the pressure inside the closed reaction process naturally rises, and the reaction can be carried out under pressurized conditions. Therefore, the fluoroalkyne compound represented by formula (1) as the target compound can be obtained with higher selectivity and higher conversion.

Thus, the closed reaction process is pressurized due to the low boiling point of the target compound, and the concentration of the substrate (starting compound) in the ether solvent increases, which can improve the reactivity. To perform the closed reaction process, it is preferable to carry out the reaction while sealing the reaction system using a batch-type pressure-resistant reaction vessel. When the reaction is carried out in a batch process, it is preferable, for example, to place a starting compound, an ether solvent, and if necessary a base etc. in a pressure vessel, such as an autoclave, raise the temperature to an appropriate reaction temperature with a heater, and carry out the reaction with stirring for a certain period of time. As the reaction atmosphere, it is preferable to carry out the reaction in an inert gas atmosphere, such as nitrogen, helium, or carbon dioxide.

In the step of performing a dehydrofluorination reaction in the present disclosure, the reaction temperature in the closed reaction process is generally preferably 0 to 400°C, more preferably 25 to 300°C, and even more preferably 50 to 200°C, from the viewpoint of the conversion in reaction and the selectivity and yield of the halogenated alkyne compound.

When no base is used or a hydroxide of an alkali metal and/or alkaline earth metal is used as a base, the reaction temperature in the closed reaction process is generally preferably 25 to 400°C, more preferably 50 to 300°C, and even more preferably 100 to 200°C, from the viewpoint of the conversion in reaction and the selectivity and yield of the halogenated alkyne compound.

When an alkoxide of an alkali metal and/or alkaline earth metal is used as a base, the reaction temperature is preferably low. This is because if the reaction temperature is too high, the addition reaction of the alkoxide to the double bond proceeds. Thus, the reaction temperature in the closed reaction process is generally preferably 0 to 200°C, more preferably 25 to 150°C, and even more preferably 50 to 100°C, from the viewpoint of the conversion in reaction and the selectivity and yield of the halogenated alkyne compound.

The reaction time is not limited, and may be a time during which the reaction can sufficiently proceed. Specifically, the reaction can proceed until there is no change in composition in the reaction system.

### Pressure-Increased Reaction Process

In the step of performing a dehydrofluorination reaction in the present disclosure, the reaction can be carried out in a pressure-increased reaction process by setting the reaction temperature to 0°C or more and the reaction pressure to more than 0 kPa. As a result, the fluoroalkyne compound represented by formula (1) as the target compound can be obtained with higher selectivity and higher conversion. When the reaction system is pressurized in this way, the concentration of the substrate (starting compound) in the ether solvent rises, which can improve the reactivity. In the pressure-increased reaction process, it is preferable to carry out the reaction while sealing the reaction system using a batch-type pressure-resistant reaction vessel. When the reaction is carried out in a batch process, it is preferable, for example, to place a starting compound, an ether solvent, and if necessary a base etc. in a pressure vessel, such as an autoclave, raise the temperature to an appropriate reaction temperature with a heater, and carry out the reaction with stirring for a certain period of time.

In the step of performing a dehydrofluorination reaction in the present disclosure, the reaction pressure is preferably more than 0 kPa. In the step of performing a dehydrofluorination reaction in the present disclosure, the reaction pressure is preferably more than 0 kPa, more preferably 5 kPa or more, even more preferably 10 kPa or more, and particularly preferably 15 kPa or more. The upper limit of the reaction pressure is not limited and is generally about 2 MPa. In the present disclosure, the pressure is gauge pressure unless otherwise specified. Here, the reaction pressure when a pressure-increased reaction process is used is described. If a pressure-increased reaction process is not used, for example, the reaction can proceed under reduced pressure or ordinary pressure.

For pressurization, the pressure in the reaction system can be increased by sending an inert gas, such as nitrogen, helium, or carbon dioxide, into the reaction system.

In the step of performing a dehydrofluorination reaction in the present disclosure, the reaction temperature in the pressure-increased reaction process is generally preferably 0 to 400°C, more preferably 25 to 300°C, and even more preferably 100 to 200°C, from the viewpoint that the elimination reaction can proceed more efficiently and the target compound can be obtained with higher selectivity, and from the viewpoint of suppressing the decrease in the conversion.

When no base is used or a hydroxide of an alkali metal and/or alkaline earth metal is used as a base, the reaction temperature in the pressure-increased reaction process is generally preferably 25 to 400°C, more preferably 50 to 300°C, and even more preferably 100 to 200°C, from the viewpoint of the conversion in reaction and the selectivity and yield of the halogenated alkyne compound.

When an alkoxide of an alkali metal and/or alkaline earth metal is used as a base, the reaction temperature is preferably low. This is because if the reaction temperature is too high, the addition reaction of the alkoxide to the double bond proceeds. Thus, the reaction temperature in the pressure-increased reaction process is generally preferably 0 to 200°C, more preferably 25 to 150°C, and even more preferably 50 to 100°C, from the viewpoint of the conversion in reaction and the selectivity and yield of the halogenated alkyne compound.

### Combination of Closed Reaction Process and Pressure-Increased Reaction Process

In the step of performing a dehydrofluorination reaction in the present disclosure, it is also possible to carry out the reaction in a continuous and pressurized reaction form, while extracting the liquid or extracting the gasified product, for example, by connecting a back pressure valve to a continuous stirred-tank reactor (CSTR).

After the dehydrofluorination reaction is completed, purification can be optionally performed according to a conventional method, thereby obtaining a fluoroalkyne compound represented by formula (1).

### (1-6) Target Compound (Halogenated Alkyne Compound)

The target compound of the present disclosure obtained in the above manner is a fluoroalkyne compound represented by formula (1):

R¹C≡CR² (1)

wherein R¹ and R² are the same or different and each is a fluorine atom or a fluoroalkyl group.

R¹ and R² in formula (1) correspond to R¹ and R² in formula (2) described above, respectively. Accordingly, specific examples of the fluoroalkyne compound represented by formula (1) to be produced include CF≡CF, CF₃C≡CF, CF₃C≡CCF₃, CF₃CF₂C≡CCF₃, CF₃CF₂C≡CCF₂CF₃, and the like.

According to the production method of the present invention, the fluoroalkyne compound represented by formula (1) may not be obtained alone, and may be obtained in the form of a composition containing the fluoroalkyne compound represented by formula (1). Even in this case, most of the compounds other than the fluoroalkyne compound represented by formula (1) are fluoroalkene compounds represented by formula (4):

R¹CF=CHR² (4)

wherein R¹ and R² are as defined above, in which only 1 mol of hydrogen fluoride is eliminated from the fluoroalkane compound represented by formula (2). Thus, it is possible to further improve the selectivity and yield of the fluoroalkyne compound represented by formula (1) by repeating the above step of performing a dehydrofluorination reaction.

For example, in Example 1 described later, CF₃CFHCFHCF₃ was used as a starting compound, and CF₃C≡CCF₃ was obtained in a yield of 49.9 mol% (conversion: 97.9% × selectivity: 51.00%), and CF₃CF=CHCF₃ was obtained in a yield of 41.7 mol% (conversion: 97.9% × selectivity: 42.62%).

In the present disclosure, when the step of performing a dehydrofluorination reaction is repeated, that is, when the number of steps is increased, CF₃CF=CHCF₃ obtained as an impurity is used as a starting material, and a dehydrofluorination reaction is performed in the same manner. Thus, if the yield of CF₃CF=CHCF₃→CF₃C≡CCF₃ in the second step is also 49.9 mol%, CF₃C≡CCF₃ is obtained in an amount that is 70.7 mol% of CF₃CFHCFHCF₃, which is the starting material, in total in the two steps.

When the first step is CF₃CFHCFHCF₃→CF₃CF=CHCF₃ and the second step is CF₃CF=CHCF₃→CF₃C≡CCF₃ as in the conventional methods, if the yield in the first step is 100 mol%, CF₃C≡CCF₃ is obtained in an amount that is 49.9 mol% of CF₃CFHCFHCF₃, which is the starting material, in the two steps since the yield in the second step is 49.9 mol% as described above.

As described above, according to the present disclosure, it is possible to obtain a specific fluoroalkyne compound using a specific fluoroalkane compound as a starting compound by only a single step, and it is possible to improve the yield of the fluoroalkyne compound when the dehydrofluorination reaction is repeated a plurality of times, compared with the conventional methods. In any case, it can be understood that the production method of the present disclosure is useful.

The thus-obtained fluoroalkyne compound can be effectively used for various applications, such as etching gases for forming cutting-edge microstructures in semiconductors, liquid crystals, etc., as well as cleaning gases, deposit gases, refrigerants, heat transfer media, and building blocks for organic synthesis. The deposit gas and the building block for organic synthesis are described later.

### 2. Method for Producing Fluoroalkyne Compound (Second Embodiment)

The method for producing a fluoroalkyne compound in the second embodiment of the present disclosure is a method for producing a fluoroalkyne compound represented by formula (1):

R¹C≡CR² (1)

wherein R¹ and R² are the same or different and each is a fluorine atom or a fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of a solvent containing an ether,
(II) the ether being a chain ether, and the dehydrofluorination reaction being performed in the presence of the solvent containing a chain ether and a base comprising a hydroxide and/or alkoxide of an alkali metal and/or alkaline earth metal.

That is, the method for producing a fluoroalkyne compound in the second embodiment of the present disclosure is a method for producing a fluoroalkyne compound represented by formula (1):

R¹C≡CR² (1)

wherein R¹ and R² are the same or different and each is a fluorine atom or a fluoroalkyl group,
the method comprising subjecting a fluoroalkane compound represented by formula (2):

R¹CHX¹CFX²R² (2)

wherein R¹ and R² are as defined above; and X¹ is a fluorine atom and X² is a hydrogen atom, or X¹ is a hydrogen atom and X² is a fluorine atom, to a dehydrofluorination reaction in the presence of a solvent containing a chain ether and a base comprising a hydroxide and/or alkoxide of an alkali metal and/or alkaline earth metal.

According to the present disclosure, by performing a dehydrofluorination reaction of the fluoroalkane compound represented by formula (2), the fluoroalkyne compound represented by formula (1) in which 2 mol of hydrogen fluoride is eliminated per mol of the fluoroalkane compound represented by formula (2) can be obtained in only a single step.

### (2-1) Starting Compound (Fluoroalkane Compound)

The fluoroalkane compound as a substrate that can be used in the production method of the present disclosure (the second embodiment) is a fluoroalkane compound represented by formula (2):

R¹CHX¹CFX²R² (2)

wherein R¹ and R² are the same or different and each is a fluorine atom or a fluoroalkyl group; and X¹ is a fluorine atom and X² is a hydrogen atom, or X¹ is a hydrogen atom and X² is a fluorine atom, as described above.

That is, the fluoroalkane compound represented by formula (2) encompasses formulas (2A) and (2B):

R¹CFHCFHR² (2A)

R¹CH₂CF₂R² (2B)

wherein R¹ and R² are the same or different and each is a fluorine atom or a fluoroalkyl group.

In formula (2), the fluoroalkyl group represented by each of R¹ and R² refers to a group formed by replacing one or more hydrogen atoms of an alkyl group with a fluorine atom, and also includes a perfluoroalkyl group formed by replacing all the hydrogen atoms of an alkyl group. The fluoroalkyl group may be a linear fluoroalkyl group or a branched fluoroalkyl group. Examples include C₁-C₁₀ fluoroalkyl groups, such as monofluoromethyl, difluoromethyl, trifluoromethyl, monofluoroethyl, difluoroethyl, trifluoroethyl, tetrafluoroethyl, pentafluoroethylfluoroethyl, monofluoropropyl, difluoropropyl, trifluoropropyl, tetrafluoropropyl, pentafluoropropyl, hexafluoropropyl, and heptafluoropropyl. From the viewpoint of producing the fluoroalkyne compound in particular with high conversion, yield, and selectivity, the fluoroalkyl group is preferably a C₁-C₄ fluoroalkyl group, and more preferably a C₁-C₃ fluoroalkyl group.

In the fluoroalkane compound as a substrate, R¹ and R² are preferably fluoroalkyl groups, more preferably perfluoroalkyl groups, and even more preferably trifluoromethyl groups, from the viewpoint of producing the fluoroalkyne compound in particular with high conversion, yield, and selectivity.

R¹ and R² may be the same or different.

Specific examples of the fluoroalkane compound as a substrate that satisfies the above conditions include CF₂HCF₂H, CF₃CFHCF₂H, CF₃CFHCFHCF₃, CF₃CF₂CFHCFHCF₃, CF₃CF₂CFHCFHCF₂CF₃, CFH₂CF₃, CF₃CH₂CF₃, CF₃CH₂CF₂CF₃, CF₃CF₂CH₂CF₂CF₃, CF₃CF₂CH₂CF₂CF₂CF₃, and the like. These fluoroalkane compounds can be used singly or in a combination of two or more. Such fluoroalkane compounds can be known or commercially available products.

### (2-2) Dehydrofluorination Reaction

The "dehydrofluorination reaction" can be the same as the "(1-2) Dehydrofluorination Reaction" described above. Various conditions can also be the same as those described above.

### (2-3) Solvent Containing Chain Ether

In the present disclosure, the reaction is performed in the presence of a solvent containing a chain ether to obtain a fluoroalkyne compound from a fluoroalkane compound by a dehydrofluorination reaction, preferably in a single step.

However, the water content is preferably small so that the fluoroalkyne compound can be easily obtained from the fluoroalkane compound by a dehydrofluorination reaction. From such a viewpoint, the water concentration of the solvent containing a chain ether is preferably 500 mass ppm or less, more preferably 400 mass ppm or less, and even more preferably 300 mass ppm or less, based on the total amount of the solvent containing a chain ether taken as 100 mass%. The lower limit of the water concentration of the solvent containing a chain ether is not limited, and is preferably 0.01 mass ppm or more from the viewpoint that it can be easily achieved technically.

In addition, to easily obtain the fluoroalkyne compound from the fluoroalkane compound by a dehydrofluorination reaction, the number of ether bonds in the chain ether is preferably as small as possible. From such a viewpoint, the number of ether bonds in the chain ether is preferably 1 to 10, more preferably 1 to 5, even more preferably 1 to 3, and particularly preferably 1 to 2.

The chain ether in the solvent containing a chain ether that satisfies the above conditions includes not only an ether compound having no cyclic structure, but also an ether compound containing a cyclic structure that does not contain an ether bond. That is, the chain ether is a concept that does not include an ether compound in which an ether bond constitutes a cyclic structure, such as tetrahydrofuran, but includes an ether compound containing a cyclic structure that does not contain an ether bond, such as diphenyl ether. When an ether compound having no cyclic structure is used, the yield of the fluoroalkyne compound as the target product can be particularly improved. When an ether compound containing a cyclic structure that does not contain an ether bond is used, the yield of impurities (hydrofluorocarbon compounds other than fluoroalkane compounds, fluoroalkene compounds, and fluoroalkyne compounds) can be particularly reduced. Specific preferred examples of such chain ethers include diethyl ether, diisopropyl ether, di(n-butyl) ether, diphenyl ether, 1,2-dimethoxyethane, 1,2-diethoxyethane, diglyme, and the like. The chain ether solvents can be used singly or in a combination of two or more. In particular, from the viewpoint of conversion, selectivity, and yield, diethyl ether, diisopropyl ether, di(n-butyl) ether, diphenyl ether, 1,2-dimethoxyethane, 1,2-diethoxyethane, and the like are preferred, diethyl ether, diisopropyl ether, di(n-butyl) ether, diphenyl ether, and the like are more preferred, and diisopropyl ether, di(n-butyl) ether, diphenyl ether, and the like are even more preferred.

In the present disclosure, the chain ether may be used in combination with other solvents, such as carbonate ester solvents such as dimethyl carbonate, ethyl methyl carbonate, diethyl carbonate, methyl propyl carbonate, and ethyl propyl carbonate; ester solvents such as ethyl acetate, propyl acetate, butyl acetate, methyl propionate, ethyl propionate, and butyl propionate; ketone solvents such as acetone, ethyl methyl ketone, and diethyl ketone; lactone solvents such as γ-butyrolactone, γ-valerolactone, tetrahydrofuran, and tetrahydropyran; cyclic ether solvents such as tetrahydrofuran; nitrile solvents such as acetonitrile, propionitrile, and benzonitrile; amide solvents such as N,N-dimethylformamide; and sulfone solvents such as dimethyl sulfoxide and sulfolane. However, from the viewpoint of, for example, the conversion in reaction, selectivity, and yield, the amount of other solvents for use is preferably small. Specifically, the amount of the chain ether for use is preferably 80 to 100 vol% (in particular 90 to 100 vol%), and the amount of other solvents is preferably 0 to 20 vol% (in particular 0 to 10 vol%), based on the total amount of the solvents for use in reaction taken as 100 vol%.

### (2-4) Base

From the viewpoint of conversion, selectivity, and yield, the step of subjecting a fluoroalkane compound to a dehydrofluorination reaction to obtain a fluoroalkyne compound in the present disclosure is performed in the presence of a base comprising a hydroxide and/or alkoxide of an alkali metal and/or alkaline earth metal.

From the viewpoint of the conversion in reaction and the selectivity and yield of the halogenated alkyne compound, the base is preferably at least one member selected from the group consisting of hydroxides and alkoxides of alkali metals and alkaline earth metals (i.e., at least one member selected from the group consisting of alkali metal hydroxides, alkaline earth metal hydroxides, alkali metal alkoxides, and alkaline earth metal alkoxides), preferably at least one member selected from the group consisting of hydroxides of alkali metals and alkaline earth metals and alkoxides of alkali metals (i.e., at least one member selected from the group consisting of alkali metal hydroxides, alkaline earth metal hydroxides, and alkali metal alkoxides), more preferably a hydroxide and/or alkoxide of an alkali metal (i.e., an alkali metal hydroxide and/or an alkali metal alkoxide), and even more preferably an alkali metal hydroxide. Specific examples of bases include sodium hydroxide, potassium hydroxide, magnesium hydroxide, calcium hydroxide, sodium methoxide, potassium tert-butoxide, sodium ethoxide, sodium tert-butoxide, potassium methoxide, potassium ethoxide, potassium tert-butoxide, magnesium methoxide, magnesium ethoxide, magnesium tert-butoxide, calcium methoxide, calcium ethoxide, calcium tert-butoxide, and the like. In particular, from the viewpoint of the conversion in reaction and the selectivity and yield of the halogenated alkyne compound, sodium hydroxide, potassium hydroxide, magnesium hydroxide, calcium hydroxide, sodium methoxide, sodium ethoxide, sodium tert-butoxide, potassium methoxide, potassium ethoxide, potassium tert-butoxide, and the like are preferred, sodium hydroxide, potassium hydroxide, sodium methoxide, sodium ethoxide, sodium tert-butoxide, potassium methoxide, potassium ethoxide, potassium tert-butoxide, and the like are more preferred, and sodium hydroxide, potassium hydroxide, and the like are even more preferred.

The amount of the base for use is not limited and is preferably about 1 mol per mol of the fluoroalkane compound represented by formula (2). Specifically, the amount of the base for use is preferably 1.0 to 10.0 mol, more preferably 1.5 to 5.0 mol, and even more preferably 2.0 to 3.0 mol, per mol of the fluoroalkane compound represented by formula (2).

### (2-5) Reaction Conditions and Target Compound

The "reaction conditions" can be the same as the "(1-5) Reaction Conditions" described above. The "target compound" can be the same as the "(1-6) Target Compound" described above. Various conditions can also be the same as those described above.

### 3. Composition

As described above, a fluoroalkyne compound can be obtained from a fluoroalkane compound preferably in a single step, and may also be obtained in a form of a composition containing a fluoroalkyne compound. In particular, according to the production method of the present disclosure, for example, a composition containing a fluoroalkyne compound represented by formula (1) and a fluoroalkene compound represented by formula (4), in which only 1 mol of hydrogen fluoride is eliminated from a fluoroalkane compound represented by formula (2):

R¹CF=CHR² (4)

wherein R¹ and R² are as defined above, can be produced.

R¹ and R² in formula (4) correspond to R¹ and R² in formula (2) described above, respectively. Thus, specific examples of the fluoroalkene compound represented by formula (4) include CF₂=CFH, CF₃CF=CFH, CF₃CH=CFH, CF₃CF=CHCF₃, CF₃CF₂CF=CHCF₃, CF₃CF₂CH=CFCF₃, CF₃CF₂CF=CHCF₂CF₃, and the like. The composition may contain one of these fluoroalkene compounds or a combination of two or more of these fluoroalkene compounds.

According to the production method of the present disclosure, a composition that contains a fluoroalkyne compound represented by formula (1) and a fluoroalkene compound represented by formula (4) in similar amounts and contains a small amount of other components tends to be produced. Thus, in the composition containing a fluoroalkyne compound in the present disclosure, the content of the fluoroalkyne compound represented by formula (1) is preferably 25.00 mol% or more, more preferably 30.00 mol% or more, and even more preferably 35.00 mol% or more, based on the total amount of the composition taken as 100 mol%. In the composition containing a fluoroalkyne compound in the present disclosure, the content of the fluoroalkyne compound represented by formula (1) may also be, for example, 75.00 mol% or less, 70.00 mol% or less, or 65.00 mol% or less, based on the total amount of the composition taken as 100 mol%. In the composition containing a fluoroalkyne compound in the present disclosure, the content of the fluoroalkene compound represented by formula (4) may be 25.00 mol% or more, 30.00 mol% or more, or 35.00 mol% or more, based on the total amount of the composition taken as 100 mol%. In the composition containing a fluoroalkyne compound in the present disclosure, the content of the fluoroalkene compound represented by formula (4) is also preferably 75.00 mol% or less, more preferably 70.00 mol% or less, and even more preferably 65.00 mol% or less, based on the total amount of the composition taken as 100 mol%. Furthermore, in the composition containing a fluoroalkyne compound in the present disclosure, the total content of the fluoroalkyne compound represented by formula (1) and the fluoroalkene compound represented by formula (4) is preferably 90.00 mol% or more, more preferably 91.00 mol% or more, and even more preferably 92.00 mol% or more, based on the total amount of the composition taken as 100 mol%. In the composition containing a fluoroalkyne compound in the present disclosure, the total content of the fluoroalkyne compound represented by formula (1) and the fluoroalkene compound represented by formula (4) may be 100.00 mol% or less, 99.99 mol% or less, or 99.98 mol% or less, based on the total amount of the composition taken as 100 mol%.

As described above, according to the production method of the present disclosure, hydrofluorocarbon compounds, such as trifluoromethane (R23), difluoromethane (R32), tetrafluoromethane (R14), monofluoromethane (R41), 1,2-difluoroethylene (R1132), and 1,1,2-trifluoroethylene (R1123) may be contained as components other than the fluoroalkyne compound represented by formula (1) and the fluoroalkene compound represented by formula (4) in the composition obtained. In this case, in the composition containing a fluoroalkyne compound in the present disclosure, the content of these other hydrofluorocarbon compounds may be 0.00 mol% or more, 0.01 mol% or more, or 0.02 mol% or more, based on the total amount of the composition taken as 100 mol%. In the composition containing a fluoroalkyne compound in the present disclosure, the content of these other hydrofluorocarbon compounds is preferably 10.00 mol% or less, more preferably 7.50 mol% or less, and even more preferably 5.00 mol% or less, based on the total amount of the composition taken as 100 mol%.

According to the production method of the present disclosure, a fluoroalkyne compound can be obtained in particular with high selectivity and yield compared with conventional methods for obtaining a fluoroalkyne compound from a fluoroalkane compound; and as a result, purification for obtaining a fluoroalkyne compound can be efficiently performed.

The composition containing a fluoroalkyne compound in the present disclosure can be effectively used for various applications, such as etching gases for forming cutting-edge microstructures in semiconductors, liquid crystals, etc., as well as cleaning gases, deposit gases, refrigerants, heat transfer media, and building blocks for organic synthesis, as in the fluoroalkyne compound alone.

The deposit gas is a gas that deposits an etching resistant polymer layer.

The building block for organic synthesis refers to a substance that can be a precursor of a compound having a highly reactive skeleton. For example, when the composition of the present disclosure is reacted with a fluorine-containing organosilicon compound, such as CF₃Si(CH₃)₃, it is possible to introduce a fluoroalkyl group, such as a CF₃ group, to convert it into a substance that can be a cleaner or a fluorine-containing pharmaceutical intermediate.

Although embodiments of the present disclosure are described above, various changes in form and details can be made without departing from the spirit and scope of the claims.

### Examples

The features of the present disclosure are clarified below while showing Examples. The present disclosure is not limited to these Examples.

In the methods for producing a fluoroalkyne compound of Examples 1 to 6 and Comparative Example 1, the starting compound was a fluoroalkane compound represented by formula (2) wherein R¹ and R³ are trifluoromethyl groups, and a fluoroalkyne compound was obtained by a dehydrofluorination reaction according to the following reaction formula:

CF₃CFHCFHCF₃ → CF₃C≡CCF₃ + 2HF

### Examples 1 to 6 and Comparative Example 1

Sodium hydroxide (NaOH), potassium hydroxide (KOH), sodium tert-butoxide (t-BuONa), or potassium tert-butoxide (t-BuOK) as a base, and di(n-butyl) ether (Bu₂O), diphenyl ether (Ph₂O), diglyme, or water as a solvent were added to an autoclave, and a starting compound (CF₃CFHCFHCF₃) was further added. The autoclave was closed with the lid to form a closed system. The concentration of the base added in the solvent was 2.5 mol/L. At this stage, the amount of the base for use was adjusted to 2 mol per mol of the starting compound. The pressure at that time was 0 kPa. Thereafter, the mixture was heated to 100°C or 150°C and stirred to allow a reaction to proceed. After the dehydrofluorination reaction was started, sampling was suitably performed, and the reaction was considered to have completed when there was no change in composition in the reaction system. The pressure at the completion of the reaction was 500 kPa.

After stirring was stopped, the reaction mixture was cooled to room temperature. Mass spectrometry was performed by gas chromatography (Shimadzu Corporation, trade name: GC-2014) according to gas chromatography-mass spectrometry (GC/MS), and structural analysis was performed by using an NMR (JEOL Ltd., trade name: 400YH) based on an NMR spectrum. From the results of mass spectrometry and structural analysis, CF₃C≡CCF₃ was confirmed to have formed as a target compound. Table 1 shows the results. In Table 1, R23 denotes trifluoromethane, R1132 denotes 1,2-difluoroethylene, PF2B denotes CF₃C≡CCF₃, which is the target compound, and 1327myz denotes CF₃CF=CHCF₃.

**Table 1**

| Run | Solvent | | Temperature Conversion | | | Sele ctivity [%] | | | | |
|---|---|---|---|---|---|---|---|---|---|---|
| | | | Base | [°C] | [%] | R23 | R1132 | PF28 | 1327myz | Others |
| Ex. 1 | | Bu2O | KOH | 150 | 97.9 | 2.94 | 1.53 | 51.00 | 42.62 | 1.9 |
| Ex. 2 | | Bu2O | NaOH | 150 | 97.6 | 2.55 | 1.26 | 51.40 | 43.60 | 1.2 |
| Ex. 3 | | Diglyme | KOH | 150 | 98.5 | 1.16 | 0.25 | 25.17 | 73.12 | 0.3 |
| Ex. 4 | | Ph2O | KOH | 150 | 99.3 | 0.88 | 0.45 | 47.17 | 49.82 | 1.7 |
| Ex. 5 | | Bu2O | t-BuOK | 100 | 99.5 | 0.037 | 0.034 | 32.5 | 67.2 | 0.3 |
| Ex. 6 | | Bu2O | t-BuONa | 100 | 99.5 | 0.22 | 0.09 | 34.03 | 64.64 | 1.0 |
| Comp. Ex. 1 | | Water | KOH | 150 | 96.3 | 1.24 | 1.55 | 0.30 | 95.40 | 1.5 |

### Example 7 and Comparative Example 2

Under the etching conditions of ICP (Inductive Coupled Plasma) discharge power: 800 W, bias power: 100 W, pressure: 3 mmTorr (0.399 Pa), electron density: 8 × 10¹⁰ to 2 × 10¹¹ cm⁻³, and electron temperature: 5 to 7 eV, an SiO₂ film and a resist film were etched using the composition (containing 51.00 mol% of CF₃C≡CCF₃ and 42.62 mol% of CF₃CF=CHCF₃) obtained in Example 1, and the etching rate and the resist selectivity (etching rate of SiO₂ film/etching rate of resist) were measured (Example 7). The same experiment was performed, except that the only gas used was CF₃C≡CCF₃ (Comparative Example 2).

Table 2 shows the results.

**Table 2**

| | Gas | Resist Selectivity |
|---|---|---|
| Example 7 | Mixed gas of CF₃CCCF₃ and CF₃CFCHCF₃ | 5.0 |
| Comparative Example 2 | Only CF₃CCCF₃ | 2.1 |

## Claims

1. A method for producing a fluoroalkyne compound represented by formula (1):
R¹C≡CR² (1)
wherein R¹ and R² are the same or different and each is a fluorine atom or a fluoroalkyl group,
the method comprising subjecting a fluoroalkane compound represented by formula (2):
R¹CHX¹CFX²R² (2)
wherein R¹ and R² are as defined above; and X¹ is a fluorine atom and X² is a hydrogen atom, or X¹ is a hydrogen atom and X² is a fluorine atom, to a dehydrofluorination reaction in the presence of a solvent containing an ether,
the method satisfying at least one requirement selected from the group consisting of the following:
(I) the fluoroalkyl group is a C₁-C₄ fluoroalkyl group, and
(II) the ether is a chain ether, and the dehydrofluorination reaction is performed in the presence of the solvent containing a chain ether, and a base comprising a hydroxide and/or alkoxide of an alkali metal and/or alkaline earth metal.

2. The production method according to claim 1, wherein the water concentration of the solvent containing an ether is 0.01 to 500 mass ppm based on the total amount of the solvent containing an ether taken as 100 mass%.

3. The production method according to claim 1 or 2,
wherein the number of ether bonds in the ether is 1 to 10.

4. The production method according to any one of claims 1 to 3, which satisfies requirement (I).

5. The production method according to claim 4, wherein the fluoroalkyl group is represented by formula (3):
-CF₂R³ (3)
wherein R³ is a fluorine atom or a C₁-C₃ fluoroalkyl group.

6. The production method according to claim 4 or 5, wherein the dehydrofluorination reaction is performed in the presence of a base.

7. The production method according to claim 6, wherein the base is a hydroxide and/or alkoxide of an alkali metal and/or alkaline earth metal.

8. The production method according to any one of claims 1 to 3, which satisfies requirement (II).

9. The production method according to claim 8, wherein the fluoroalkyl group has 1 to 10 carbon atoms.

10. The production method according to claim 8 or 9, wherein the fluoroalkyl group is represented by formula (3):
-CF₂R³ (3)
wherein R³ represents a fluorine atom or a C₁-C₉ fluoroalkyl group.

11. The production method according to any one of claims 1 to 10, wherein the reaction temperature in the dehydrofluorination reaction is 0 to 300°C.

12. A composition comprising a fluoroalkyne compound represented by formula (1):
R¹C≡CR² (1)
wherein R¹ and R² are the same or different and each is a fluorine atom or a C₁-C₄ fluoroalkyl group, and a fluoroalkene compound represented by formula (4):
R¹CF=CHR² (4)
wherein R¹ and R² are as defined above,
the content of the fluoroalkyne compound represented by formula (1) being 25.00 to 75.00 mol%, the content of the fluoroalkene compound represented by formula (4) being 25.00 to 75.00 mol%, and the total content of the fluoroalkyne compound represented by formula (1) and the fluoroalkene compound represented by formula (4) being 90.00 to 100.00 mol%, based on the total amount of the composition taken as 100 mol%.

13. The composition according to claim 12, which is for use as a cleaning gas, an etching gas, a refrigerant, a heat transfer medium, or a building block for organic synthesis.
